# EUROPÄISCHE PATENTSCHRIFT

(11) **EP 2 514 353 B1**
(45) Veröffentlichungstag und Bekanntmachung des Hinweises auf die Patenterteilung: **01.06.2016**
(21) Anmeldenummer: 12158629.1
(22) Anmeldetag: 08.03.2012
(51) Int. Cl.: A61B 1/00, G02B 23/24, A61B 1/002

(54) **Endoskop mit variabler Blickrichtung**
Endoscope with variable view angle
Endoscope à direction d'observation variable

(30) Priorität: 15.04.2011 DE 102011007484
(43) Veröffentlichungstag der Anmeldung: 24.10.2012
(73) Patentinhaber: Henke-Sass, Wolf GmbH, 78532 Tuttlingen (DE)
(72) Erfinder: Meyer, Andrea, 72365 Ratshausen (DE); Häckl, Norbert, 88637 Leibertingen (DE)
(74) Vertreter: Patentanwälte Geyer, Fehners & Partner mbB

(56) Entgegenhaltungen:
- EP-A2- 1 166 710
- WO-A1-01/39657
- DE-A1- 2 328 595

## Beschreibung

Die vorliegende Erfindung betrifft ein Endoskop mit variabler Blickrichtung gemäß Anspruch 1.

Dabei ist das Endoskop in der Regel für einen möglichen Schwenkbereich der Umlenkeinheit mit einer ersten und einer zweiten Grenze ausgelegt, so daß bei Schwenkstellungen innerhalb dieses Bereichs optimale Abbildungsverhältnisse herrschen. Diese optimalen Abbildungsverhältnisse liegen jedoch nicht mehr vor, wenn die obere oder untere Grenze des Schwenkbereichs überschritten wird. In diesem Fall können beispielsweise nachteilige Abschattungseffekte, unerwünschtes Streulicht, verschlechterte Abbildungen, etc. auftreten. Nachdem die Mechanik zum Verschwenken der Umlenkeinheit immer ein gewisses Spiel aufweisen kann, kann es in nachteiliger Weise zu verschlechterten Abbildungseigenschaften kommen, wenn als Schwenkstellung die obere oder untere Grenze des Schwenkbereiches eingestellt und aufgrund des Spieles der Schwenkmechanik während der Benutzung des Endoskopes überschritten wird.

Aus der WO 01/39657 A1 ist ein Endoskop mit den Merkmalen des Oberbegriffs des Anspruches 1 bekannt. Die DE 2 328 595 beschreibt ein Endoskop mit einem schwenkbar gelagerten Prisma am distalen Ende des Endoskopschaftes, um die Blickrichtung einzustellen, wobei eine axial verschiebbare Abschirmplatte vorgesehen ist, die gelenkig mit einem Halterahmen des Prismas verbunden ist.

Ausgehend hiervon ist es Aufgabe der Erfindung, ein Endoskop mit variabler Blickrichtung bereitzustellen, bei dem die oben beschriebenen Schwierigkeiten möglichst nicht mehr auftreten.

Die Aufgabe wird bei einem Endoskop der eingangs genannten Art durch das Vorsehen des Merkmals des kennzeichnenden Teils des Anspruches 1 gelöst.

Da bei dem erfindungsgemäßen Endoskop der erste mechanische Anschlag vorgesehen ist, an dem die Umlenkeinheit bei Erreichen der ersten Grenze anliegt, kann sicher ein Überschreiten der ersten Grenze verhindert werden, selbst wenn z.B. aufgrund des Spiels der Umlenkmechanik ein über der Grenze liegender Schwenkwinkel der Umlenkeinheit auftreten könnte. Gleichzeitig kann das Endoskop kompakt ausgebildet werden, da durch Ausbildung des ersten Anschlags am distalen Ende eines verschiebbar im Endoskopschaft angeordneten Schiebelementes platzsparend der erste Anschlag vorgesehen werden kann.

Bevorzugt weist das Endoskop einen zweiten mechanischen Anschlag auf, an dem die Umlenkeinheit bei Erreichen der zweiten Grenze anliegt. Damit wird sichergestellt, daß weder die erste, noch die zweite Grenze des Schwenkbereiches in unerwünschter Weise überschritten wird. Die Abbildungsqualität des erfindungsgemäßen Endoskops kann somit für alle eingestellten, im Schwenkbereich liegenden Schwenkstellungen sichergestellt werden.

Der zweite Anschlag kann an einer Fassung eines zur Umlenkeinheit benachbarten optischen Elementes der Abbildungsoptik ausgebildet sein. Dabei handelt es sich bevorzugt um die Fassung des direkt zur Umlenkeinheit benachbarten optischen Elementes der Abbildungsoptik.

Insbesondere kann das Schiebeelement als Rohr ausgebildet sein (es kann dann z.B. auch als Zugrohr bezeichnet werden). Dies erleichtert die Herstellung des Endoskops.

Ferner kann das distale Ende des Schiebeelementes einen Abschirmungsabschnitt aufweisen, wobei das distale Ende des Schiebeelementes so mit der Umlenkeinheit gekoppelt ist, daß eine Verschiebung des Schiebeelementes eine Änderung der Schwenkstellung der Umlenkeinheit und gleichzeitig eine Verschiebung des Abschirmungsabschnittes bewirkt, um zeitgleich mit der Einstellung der gewünschten Blickrichtung eine Streulichtabschirmung für den Bereich zwischen der Umlenkeinheit und einem der Umlenkeinheit nachfolgenden Optikelemente der Abbildungsoptik zu erreichen.

Da das distale Ende des Schiebeelements den Abschirmungsabschnitt aufweist, wird zeitgleich mit Einstellung der Blickrichtung auch der Abschirmungsabschnitt und somit die Blende zur Unterdrückung von unerwünschtem Streulicht positioniert, so daß ohne Zeitverzögerung das Endoskop bei Änderung der Blickrichtung benutzt werden kann. Insbesondere ist kein manuelles Nachführen der Blendenposition nach Einstellung einer Blickrichtung notwendig.

Bevorzugt greift das distale Ende des Schiebeelementes zum Verschwenken der Umlenkeinheit in einem Bereich an, der nicht auf der Schwenkachse der Umlenkeinheit liegt. Ferner kann zur Kopplung des distalen Endes des Schiebeelementes mit der Umlenkeinheit ein Betätigungszapfen als erstes Verbindungselement und eine den Betätigungszapfen führende Aufnahme als zweites Verbindungselement vorgesehen sein, wobei eines der beiden Verbindungselemente an der Umlenkeinheit und das andere der beiden Verbindungselemente am Schiebeelement vorgesehen ist. Bevorzugt ist der Betätigungszapfen an der Umlenkeinheit vorgesehen. Die Umlenkeinheit weist bevorzugt ein in einem Halter positioniertes Umlenkelement (z.B. einen Spiegel oder ein Prisma) auf. In diesem Fall ist der Betätigungszapfen bevorzugt am Halter vorgesehen.

Bei dem erfindungsgemäßen Endoskop kann beim Erreichen der ersten und/oder zweiten Grenze jeweils ein Teil des Halters am entsprechenden Anschlag anliegen. Damit wird in einfacher Art und Weise ein mechanischer Stopp realisiert, mit dem verhindert werden kann, daß die erste bzw. zweite Grenze in unerwünschter Weise überschritten wird.

Insbesondere kann das Schiebeelement die Umlenkeinheit antreiben, so daß eine einfach zu realisierende mechanische Lösung vorliegt, bei der synchron mit Einstellung der Blickrichtung der Abschirmungsabschnitt zur Unterdrückung von Streulicht positioniert wird.

Die Drehachse des Halters kann bevorzugt senkrecht zur Längsrichtung des Endoskopschaftes liegen. Der Halter kann mittels des Schiebeelementes um seine Drehachse gedreht werden. Dazu kann das proximale Ende des Schiebeelementes mittels des Betätigungselementes axial bewegt werden. Insbesondere kann das Betätigungselement über ein erstes Getriebe mit dem Schiebeelement mechanisch verbunden sein. Das erste Getriebe kann bevorzugt so ausgebildet sein, daß es eine Drehbewegung des Betätigungselementes in eine axiale Bewegung des Schiebeelementes umsetzt.

Das distale Ende des Schiebeelementes weist den Abschirmungsabschnitt auf, der eine Streulichtblende bildet, die unabhängig von der eingestellten Schwenkstellung des Halters des Umlenkelementes sicherstellt, daß kein unerwünschtes Streulicht zwischen dem Umlenkelement bzw. der Umlenkeinheit und dem nachfolgenden Optikelement in der Abbildungsoptik gelangt.

Dazu ist das distale Ende des Schiebeelementes bevorzugt direkt mit dem Halter des Umlenkelementes mechanisch gekoppelt.

Bei dem erfindungsgemäßen Endoskop kann eine erste Markierungsblende vorgesehen sein, die mechanisch mit dem Betätigungselement so gekoppelt ist, daß sie synchron mit einer Änderung der Schwenkstellung der Umlenkeinheit bewegt wird und mittels ihrer sichtbaren Lage bei Betrachtung des Bildes die eingestellte Blickrichtung anzeigt. Somit kann auf einfache Art und Weise im Bild immer die eingestellte Blickrichtung dargestellt werden. Einem Benutzer kann daher bei Betrachtung des Bildes stets auch visuell die Information bereitgestellt werden, welche Blickrichtung gerade eingestellt ist.

Das Betätigungselement ist bevorzugt drehbar am Handgriff gelagert. Insbesondere ist es drehbar um die Längsachse des Endoskopschaftes gelagert. Dies erleichtert die Bedienung des Endoskopes.

Ferner kann bei dem erfindungsgemäßen Endoskop der Endoskopschaft drehbar am Handgriff gelagert sein und das Endoskop eine zweite Markierungsblende aufweisen, die drehfest mit dem Endoskopschaft verbunden ist und die mittels ihrer sichtbaren Lage bei Betrachtung des Bildes die Drehstellung des Endoskopschaftes anzeigt. Somit werden in vorteilhafter Weise dem Betrachter visuell sowohl die Blickrichtung des Endoskopes als auch die Drehstellung des Endoskopschaftes dargeboten, wenn er das Bild der Abbildungsoptik betrachtet.

Insbesondere kann über die Lage der ersten Markierungsblende relativ zur Lage der zweiten Markierungsblende bei Betrachtung des Bildes die eingestellte Blickrichtung angezeigt werden. So kann z.B. der Winkelabstand der beiden Markierungsblenden variiert werden, um die Blickrichtung anzuzeigen. Insbesondere kann der Winkelabstand der beiden Markierungsblenden der eingestellten Blickrichtung (beispielsweise bezogen auf die Längsrichtung des Endoskopschaftes) entsprechen. Somit ist für den Betrachter in einfachster Art und Weise zu erfassen, welche Blickrichtung gerade eingestellt ist.

Bei dem erfindungsgemäßen Endoskop kann das Betätigungselement mit der ersten Markierungsblende über ein Untersetzungsgetriebe verbunden sein. Damit wird in vorteilhafter Weise erreicht, daß die Bewegung (beispielsweise Drehung) der ersten Markierungsblende geringer ist als die entsprechende Bewegung des Betätigungselementes.

Das Untersetzungsgetriebe kann eine Drehung des Betätigungselementes in eine axiale Bewegung und die axiale Bewegung in eine Drehbewegung der ersten Markierungsblende umsetzen. Dies kann mechanisch mit der gewünschten Genauigkeit leicht hergestellt werden.

Bevorzugt ist die erste Markierungsblende im Bereich der Feldblende der Abbildungsoptik positioniert. Auch die zweite Markierungsblende kann bevorzugt im Bereich der Feldblende der Abbildungsblende positioniert sein.

Die erste Markierungsblende ist bevorzugt relativ zur Abbildungsoptik drehbar gelagert.

Insbesondere kann die erste Markierungsblende an einer drehbar gelagerten Hülse, die z.B. koaxial zur Längsrichtung bzw. -achse des Endoskopschaftes angeordnet ist, ausgebildet sein.

Das erfindungsgemäße Endoskop kann ein Endoskop mit einem starren Endoskopschaft sein. Es ist jedoch auch möglich, daß der Endoskopschaft zumindest abschnittsweise abwinkelbar ausgebildet ist.

Das distale Ende des Endoskopschaftes kann mit einem Deckglas verschlossen sein. Insbesondere kann das distale Ende hermetisch dicht verschlossen sein, so daß der Endoskopschaft autoklavierbar ist.

Bei dem schwenkbaren Umlenkelement handelt es sich bevorzugt um einen Umlenkspiegel oder ein Umlenkprisma.

In dem Endoskopschaft ist bevorzugt noch ein Beleuchtungskanal vorgesehen, über den das abzubildende Objekt beleuchtet werden kann. Die Beleuchtung kann beispielsweise mittels Lichtleitfasern erfolgen, die am Handgriff mit Licht beaufschlagt werden können. Natürlich sind auch andere Arten der Beleuchtung möglich. Insbesondere kann eine Lichtquelle (z.B. eine oder mehrere LED-Dioden) am distalen Ende des Endoskopschaftes zur Beleuchtung vorgesehen sein.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Perspektivdarstellung einer Ausführungsform des erfindungsgemäßen Endoskopes;
- Fig. 2: eine vergrößerte Schnittdarstellung des distalen Endes des Endoskopschaftes von Fig. 1;
- Fig. 3: eine vergrößerte Seitenansicht des distalen Endes des Endoskopschaftes mit einer ersten Schwenkstellung des Umlenkprismas;
- Fig. 4: eine Draufsicht des distalen Endes des Endoskopschaftes gemäß Fig. 3;
- Fig. 5: eine vergrößerte Seitenansicht des distalen Endes des Endoskopschaftes mit einer zweiten Schwenkstellung des Umlenkprismas;
- Fig. 6: eine Draufsicht des distalen Endes des Endoskopschaftes gemäß Fig. 5;
- Fig. 7: eine vergrößerte Schnittansicht des distalen Endes des Endoskopschaftes gemäß Figur 5;
- Fig. 8: eine vergrößerte Schnittansicht des distalen Endes des Endoskopschaftes gemäß Figur 3;
- Fig. 9: eine vergrößerte Schnittansicht des proximalen Endes des Handgriffes des Endoskops gemäß Fig. 1;
- Fig. 10 und 11: Darstellungen zur Erläuterung der festen Markierungsblende;
- Fig. 12 und 13: Darstellungen zur Erläuterung der festen und drehbaren Markierungsblende;
- Fig. 14: eine vergrößerte Schnittdarstellung des rechten Abschnitts von Fig. 9 ohne Betätigungselement;
- Fig. 15: eine Darstellung zur Erläuterung der festen und drehbaren Markierungsblende;
- Fig. 16: eine perspektivische Explosionsdarstellung der beiden Markierungshülsen;
- Fig. 17: eine schematische Ansicht einer Variante der festen Markierungshülse;
- Fig. 18: eine schematische Darstellung einer Variante der drehbaren Markierungshülse, und
- Fig. 19: eine schematische Ansicht zur Erläuterung der Art der Darstellung der beiden Markierungsblenden bei Betrachtung des Bildes der Abbildungsoptik.

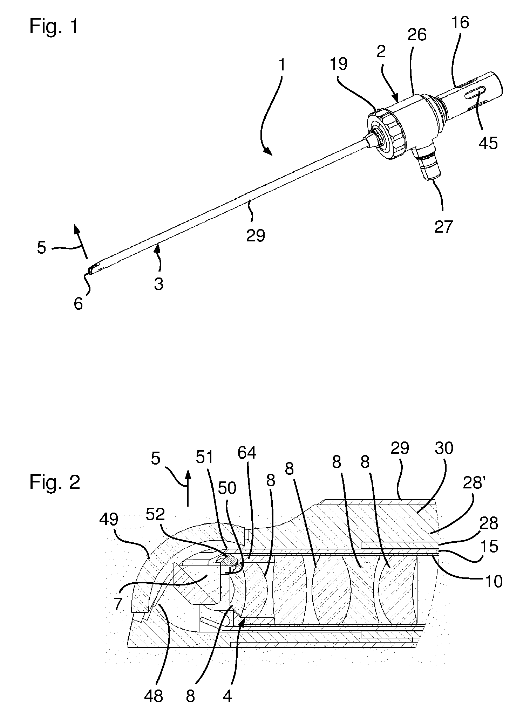

Bei der in Fig. 1 gezeigten Ausführungsform umfaßt das erfindungsgemäße Endoskop 1 mit variabler Blickrichtung einen Handgriff 2 sowie einen mit dem Handgriff 2 verbundenen Endoskopschaft 3, dessen Mantelrohr 29 in Fig. 1 sichtbar ist.

Wie insbesondere der vergrößerten Schnittdarstellung des distalen Endes 6 des Endoskopschaftes 3 in Fig. 2 zu entnehmen ist, ist im Endoskopschaft 3 eine Abbildungsoptik 4 angeordnet, mit der ein in Blickrichtung 5 der Abbildungsoptik 4 vor dem Endoskopschaft 3 befindliches Objekt als Bild abgebildet werden kann.

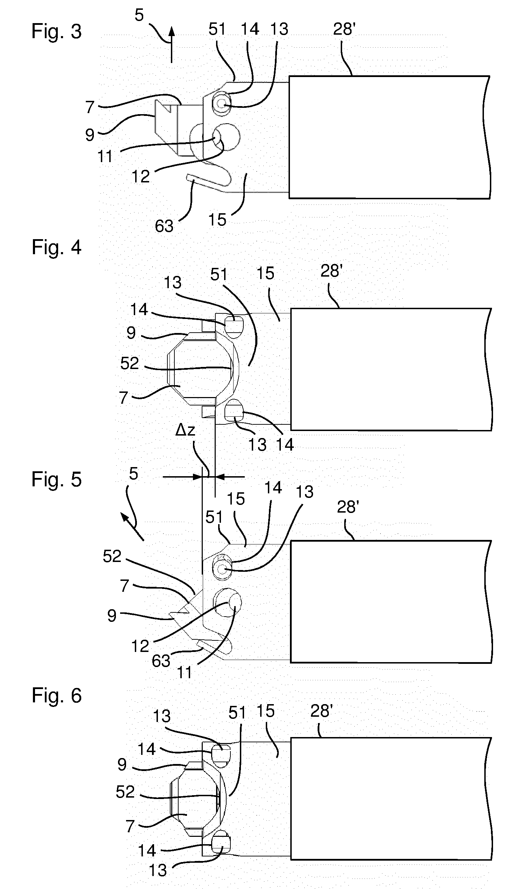

Die Abbildungsoptik 4 umfaßt ein Umlenkprisma 7 sowie diesem nachgeordnete Linsen 8. Das Umlenkprisma 7 sitzt in einem Prismenhalter 9, der schwenkbar am distalen Ende eines im Endoskopschaft 3 angeordneten Optikrohrs 10 gelagert ist, wie am besten in der vergrößerten Seitenansicht und Draufsicht des distalen Endes 6 des Endoskopschaftes 3 in Figuren 3 und 4 gezeigt ist, wobei bei den Darstellungen in Figuren 3 und 4 jeweils das Mantelrohr 29 nicht eingezeichnet ist.

Zur schwenkbaren Lagerung weist der Prismenhalter 9 zwei eine Schwenkachse bildende Lagerzapfen 11 auf, die in entsprechenden Aufnahmen 12 des distalen Endes des Optikrohrs 10 sitzen (in Fig. 3 ist nur ein Teil des linken Lagerzapfens 11 sowie ein Teil der linken Aufnahme 12 sichtbar).

Ferner weist der Prismenhalter 9 noch zwei Betätigungszapfen 13 auf, die in Aufnahmen 14 eines Zugrohrs 15 sitzen, in dem Optikrohr 10 positioniert ist, wobei das Zugrohr 15 das Optikrohr 10 in den Darstellungen von Fig. 3 und 4 nahezu vollständig überdeckt. Das Zugrohr 15 ist in Längsrichtung des Endoskopschaftes 3 relativ zum Optikrohr 10 und zum Mantelrohr 29 verschiebbar gelagert, wobei die axiale Stellung des Zugrohrs 15 mittels eines am Handgriff 2 angeordneten Betätigungselementes 16 (Fig. 1) einstellbar ist, wie nachfolgend noch im Detail beschrieben wird.

In Fig. 5 und 6 sind die gleichen Ansichten wie in Fig. 3 und 4 gezeigt, wobei jedoch das Zugrohr 15 axial verschoben ist im Vergleich zu Fig. 3 und 4. Die axiale Verschiebung ist als Δz zwischen Figuren 4 und 5 eingezeichnet. Wie ein Vergleich der Darstellungen von Fig. 3 und 4 mit denen von Fig. 5 und 6 zeigt, führt eine axiale Verschiebung des Zugrohres 15 dazu, daß sich die Betätigungszapfen 13 mit dem konstruktiv vorgegebenen Abstand zu den Lagerzapfen 11 um diese bewegen. Die Betätigungszapfen 13 werden somit auf einer in der Zeichenebene von Fig. 3 und 5 liegenden Kreisbahn bewegt, deren Mittelpunkt der Lagerzapfen 11 und somit der Durchstoßpunkt der Schwenkachse mit der Zeichenebene von Fig. 3 und 5 ist. Diese Bewegung der Betätigungszapfen 13 ist möglich, da sie in den Aufnahmen 14 des distalen Endes des Zugrohrs 15 verschiebbar gelagert sind (in Fig. 3 und 5 gesehen, von oben nach unten verschiebbar).

Somit führt die axiale Verschiebung des Zugrohrs 15 zu einem Verschwenken des Prismenhalters 9 um die durch die Lagerzapfen 11 definierte Schwenkachse, die senkrecht zur Zeichenebene in Figuren 3 und 5 verläuft, und somit zu einem Verschwenken des Umlenkprismas 7, wodurch die Blickrichtung 5 der Abbildungsoptik 4 verändert wird. So ist die Blickrichtung 5 in der Darstellung von Fig. 3 und 4 ca. 90° (bezogen auf die Längsrichtung des Endoskopschaftes 3, die jeweils in der Zeichenebene von Figuren 3 bis 6 liegt und von links nach rechts verläuft). In Fig. 5 und 6 beträgt die Blickrichtung 5 hingegen ca. 45°.

Das Zugrohr 15 bzw. das distale Ende des Zugrohrs 15 ist so ausgebildet, daß es gleichzeitig als bewegliche Streulichtblende dient, die bei jeder Schwenkstellung des Umlenkprismas 7 sicher verhindert, daß unerwünschtes Streulicht in den Bereich 50 (Fig. 2) zwischen dem Prismenhalter 9 und den Linsen 8 der Abbildungsoptik 4 gelangt, was zu einer unerwünschten Verschlechterung der Abbildungsqualität der Abbildungsoptik 4 führen würde.

Dazu weist das Zugrohr 15 einen distalen oberen Abschirmungsabschnitt 51 auf, der stets direkt oberhalb des hinteren Teils 52 des Prismenhalters 9 positioniert ist, wie in Fig. 2 und auch in den verschiedenen Schwenkstellungen gemäß Fig. 3 bis 6 schematisch dargestellt ist. Dadurch wird gewährleistet, daß unabhängig von der eingestellten Schwenkstellung des Umlenkprismas 7 kein Streulicht in den Bereich 50 gelangt.

Das Zugrohr 15 dient somit zur Abschirmung des unerwünschten Streulichtes und treibt zeitgleich das Umlenkprisma 7 zur Einstellung der gewünschten Schwenkstellung und somit zur Einstellung der gewünschten Blickrichtung 5 an. So kann nach Einstellung einer neuen Schwenkstellung ohne Zeitverzögerung das Endoskop benutzt werden, da stets das Eindringen von Streulicht sicher verhindert wird. Die Abbildungsoptik 4 ist somit stets vor direkter Streulichteinstrahlung abgeschirmt.

Durch das in Verbindung mit Figuren 3-6 beschriebene Verschwenken bzw. Drehen des Umlenkprismas 7 können Blickrichtungen im Bereich von ca. 10° und ca. 95° eingestellt werden. Somit sind die Schwenkstellungen des Umlenkprismas 7 zu den Blickrichtungen von 10° und 95° eine erste und zweite bzw. eine untere und eine obere Grenze des möglichen Schwenkbereichs des Prismenhalters 9 und somit des Umlenkprismas 7, für den das erfindungsgemäße Endoskop ausgelegt ist. Ein Überschreiten dieser Grenzen (also Blickrichtungen von < 10° oder Blickrichtungen von > 95°) kann in unerwünschter Weise zu einer verschlechterten Abbildung führen. Dies kann sich beispielsweise durch einen unerwünschten Beschnitt im abgebildeten Bild, durch Streulicht, durch schlechte Beleuchtung und/oder generell schlechte Abbildung zeigen.

Um dies zu verhindern, weist das erfindungsgemäße Endoskop einen ersten und einen zweiten Anschlag 61, 62 auf, der jeweils verhindert, daß die untere Grenze bzw. die obere Grenze des Schwenkbereichs überschritten wird.

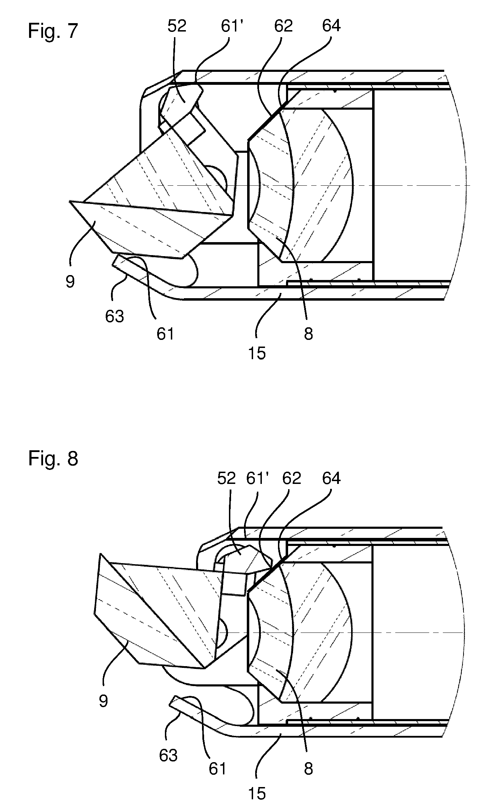

Bei der Schnittdarstellung in Figur 7 ist der erste Anschlag 61 gezeigt, der durch den distalen unteren Endbereich 63 des Zugrohres 15 gebildet ist. Genauer dient die Innenseite des distalen unteren Endbereichs 63 als erster Anschlag, an dem der Prismenhalter 9 anliegt, so daß ein weiteres Verschwenken zu kleineren Blickrichtungen als die hier vorliegende untere Grenze von ca. 10° sicher verhindert wird. Somit kann sicher verhindert werden, daß das vorliegende Spiel in der Schwenkmechanik nicht in unerwünschter Weise dazu führt, daß Blickrichtungen von kleiner als ca. 10° vorliegen. Es wird somit sicher ein Überschreiten der unteren Grenze erreicht.

Ferner kann zusätzlich (wie in Figur 7 gezeigt ist) oder alternativ der hintere Teil 52 des Prismenhalters 9 so ausgebildet sein, daß er bei einer Blickrichtung von ca. 10° an der Innenseite des oberen distalen Endes des Zugrohrs 15 anliegt, so daß dieser Bereich einen weiteren bzw. den ersten Anschlag 61' bildet.

Wie der Darstellung in Figur 8 zu entnehmen ist, bildet die Fassung 64 der Linse 8, die unmittelbar zum Umlenkprisma 7 benachbart ist, den zweiten Anschlag 62. So liegt der hintere Teil 52 des Prismenhalters 9 an der Linsenfassung 64 im Bereich 62 an, so daß ein weiteres Verschwenken des Prismenhalters 9 in Richtung zu noch größeren Blickrichtungen sicher verhindert werden kann.

Mit dem erfindungsgemäßen Endoskop ist es somit möglich, ein Überschreiten der Grenzen des bereitgestellten Schwenkbereichs sicher zu verhindern. Schwenkstellungen oder Schwenk- bzw. Drehwinkel des Prismenhalters 9 und somit des Prismas 7, die zu Blickrichtungen führen würden, die außerhalb des vorgegebenen Blickwinkelbereichs (hier ≥ 10° und ≤ 95°) liegen, können daher sicher verhindert werden. Selbst das nicht zu vermeidende Spiel in der Umlenkmechanik für das Umlenkprisma 7 führt daher nicht in nachteiliger Weise dazu, daß die obere und/oder untere Grenze des Schwenkbereichs in unerwünschter Weise überschritten wird, was z.B. zu einer verschlechterten Abbildung führen würde.

Natürlich ist der angegebene Blickwinkelbereich und der sich daraus ergebende Schwenkbereich nun beispielhaft angegeben. So kann der Blickwinkelbereich z.B. 90°, 100°, 110°, 120° oder 130° umfassen, wobei die untere Grenze bevorzugt 0° beträgt (also eine Blickrichtung in Richtung der Längsachse des Endoskopschaftes) und sich somit für die obere Grenze 90°, 100°, 110°, 120° bzw. 130° ergibt. Natürlich kann die untere Grenze auch einen Wert kleiner als 0° aufweisen, wie z.B. -5° oder -10°. Für eine untere Grenze von -5° ergeben sich für die angegebenen Blickwinkelbereiche als obere Grenze 85°, 95°, 105°, 115° bzw. 125°. Auch Werte für die untere Grenze von größer als 0°sind möglich, wie z.B. 5°, 10°oder 15°. Die konkrete Festlegung des Blickwinkelbereichs sowie der unteren und oberen Grenze und somit das konkrete Design des Endoskops wird in Abhängigkeit des jeweiligen Anwendungsfalles gewählt.

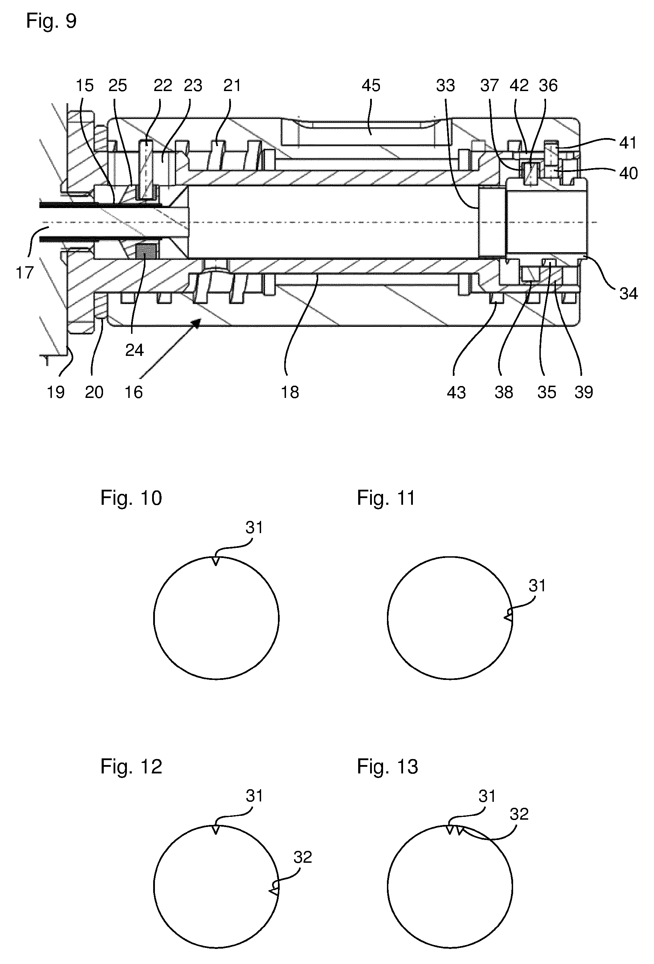

Die Abbildungsoptik 4 weist noch ein Bildübertragungssystem (hier in der Form von Stablinsen, von denen eine in der vergrößerten Schnittdarstellung des proximalen Abschnittes des Handgriffes 2 in Fig. 9 sichtbar ist) im Endoskopschaft 3 und im Handgriff 2 auf, das dazu dient, das aufgenommene Bild bis zum proximalen Ende des Handgriffes 2 zu übertragen, wo es dann bereitgestellt ist. Das bereitgestellte Bild kann direkt oder über ein proximal angeordnetes Okular betrachtet werden. Auch ist es möglich, beispielsweise eine Videokamera am proximalen Ende des Handgriffes 2 anzubringen, die das Bild aufnimmt und über eine Ausgabeeinheit (beispielsweise einen Monitor) darstellen kann.

Wie der vergrößerten Schnittdarstellung des proximalen Abschnitts des Handgriffs 2 in Fig. 9 zu entnehmen ist, ist das Betätigungselement 16 hülsenförmig ausgebildet und drehbar auf einer Führungshülse 18 gelagert, die ihrerseits drehfest mit einem Hauptteil 19 des Handgriffes 2 verbunden ist. Zwischen dem distalen Ende des Betätigungselements 16 und der Führungshülse 18 ist eine Gleitscheibe 20 vorgesehen.

Das Betätigungselement 16 weist auf seiner Innenseite im distalen Bereich eine erste schraubenförmig verlaufende Nut 21 auf, in die das obere Ende eines ersten Bolzens 22 einsteht. Der erste Bolzen 22 erstreckt sich dabei durch eine in Längsrichtung des Endoskopschaftes 3 (und somit von rechts nach links in Fig. 9) erstreckendes erstes Langloch 23 der Führungshülse 18 und ist mit seinem unteren Ende über einen ersten Teflonring 24 in einem Verbindungsteil 25 befestigt, das mit dem proximalen Ende des Zugrohrs 15 drehfest verbunden ist.

Aufgrund dieses Aufbaus führt eine Drehung des Betätigungselementes 16 um die Längsachse des Endoskopes und relativ zum Hauptteil 19 dazu, daß der erste Bolzen 22 in axialer Richtung (aufgrund der Führung durch das erste Langloch 23 in der drehfest mit dem Hauptteil 19 verbundenen Führungshülse 18) bewegt wird, so daß das Verbindungsteil 25 und somit das Zugrohr 15 axial verschoben werden. Diese Verschiebung führt am distalen Ende des Zugrohres 15, wie in Fig. 3 bis 6 dargestellt ist, dazu, daß eine gewünschte Schwenkstellung des Umlenkprismas 7 eingestellt wird.

Der Handgriff 2 umfaßt ferner ein Mittelteil 26 mit einem Lichtleiteranschluß 27 (Fig. 1). Das Mittelteil 26 ist drehfest mit dem Endoskopschaft 3 verbunden und kann relativ zum Hauptteil 19 um die Längsachse des Endoskopschaftes 3 gedreht werden. Dazu ist der Endoskopschaft 3 drehbar im Hauptteil 19 geführt.

Ferner umfaßt der Endoskopschaft 3, wie am besten aus Fig. 2 ersichtlich ist, ein Innenrohr 28 mit einem distalen Endstück 28', in denen das Zugrohr 15, das Optikrohr 10 sowie die Abbildungsoptik 4 angeordnet sind, und das Mantelrohr 29, in dem das Innenrohr 28 samt distalem Endstück 28' eingesetzt ist. Der Innendurchmesser des Mantelrohrs 29 ist größer als der Außendurchmesser des Innenrohrs 28, so daß zwischen den beiden Rohren 28 und 29 ein sich entlang der Längsrichtung des Endoskopschaftes 3 erstreckender Zwischenraum 30 vorhanden ist. In dem Zwischenraum 30 sind (in Fig. 2 nicht gezeigte) Lichtleitfasern angeordnet, die zur Beleuchtung des abzubildenden Objektes dienen. Die Lichtleitfasern können über den Lichtleiteranschluß 27 am Mittelteil 26 mit Licht beaufschlagt werden. Das distale Endstück 28' weist eine distale Öffnung 48 auf, die mittels einer Glasabdeckung 49 verschlossen ist (bevorzugt hermetisch dicht), so daß die Abbildungsoptik 4 vor Verschmutzungen geschützt ist.

Damit der Benutzer die Drehstellung des Endoskopschaftes optisch erfassen kann, wenn er das mittels der Abbildungsoptik 4 erzeugte Bild betrachtet, ist am proximalen Ende des Optikrohrs (Fig. 9) eine feste Markierungshülse 33 mit einer festen Markierungsblende 31 drehfest mit dem Optikrohr 10 verbunden. Die feste Markierungsblende 31 weist hier die Form eines Dreieckes auf, wie in Fig. 10 schematisch dargestellt ist. Wenn der Benutzer den Endoskopschaft um 90° nach rechts dreht (durch Drehen des Mittelteiles 26 relativ zum Hauptteil 19), bewegt sich die feste Markierungshülse 33 und somit die feste Markierungsblende 31 mit, so daß der Benutzer die Markierungsblende in der in Fig. 11 gezeigten Position zusammen mit dem Bild sieht.

Bei der hier beschriebenen Ausführungsform des erfindungsgemäßen Endoskop ist jedoch nicht nur eine feste Markierungsblende 31 vorgesehen, sondern zusätzlich noch eine drehbare Markierungsblende 32, die im Bild ferner die Blickrichtung 5 der Abbildungsoptik 4 und somit die Schwenkstellung des Umlenkprismas 7 anzeigt. Die drehbare Markierungsblende 32 kann die gleiche Form (beispielsweise Dreiecksform) wie die feste Markierungsblende 31 aufweisen, wie in Fig. 12 schematisch angedeutet ist. In Fig. 12 ist der Fall gezeigt, daß die Schwenkstellung des Prismas ca. 95° entspricht. Wird nun die in Fig. 5 und 6 gezeigte Schwenkstellung eingestellt (Blickrichtung von ca. 10°), ändert sich die Position der drehbaren Markierungsblende 32 relativ zur festen Markierungsblende 31, wie in Fig. 13 dargestellt ist. Somit sieht der Benutzer bei Betrachtung des Bildes über den Drehwinkel zwischen beiden Markierungsblenden 31 und 32 die eingestellte Schwenkstellung des Umlenkprismas und daher die eingestellte Blickrichtung 5. Natürlich sind die beiden Markierungsblenden 31 und 32 bevorzugt so ausgebildet, daß sie eindeutig unterscheidbar sind. So können die beiden Markierungsblenden 31 und 32 unterschiedliche Farben aufweisen. Zusätzlich oder alternativ können die beiden Markierungsblenden 31, 32 unterschiedliche Formen besitzen.

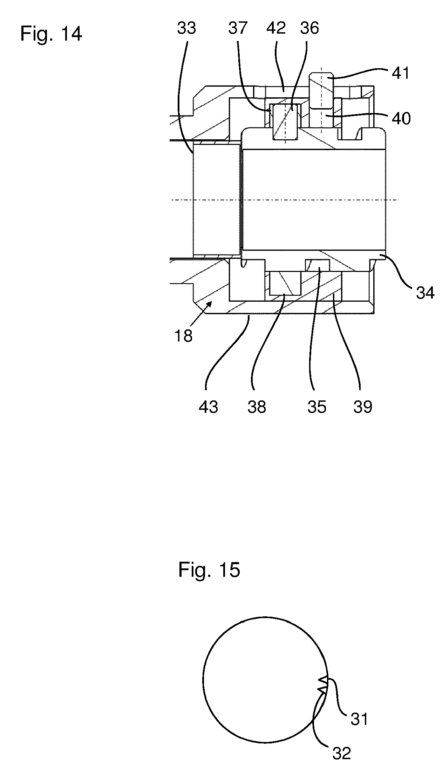

Die drehbare Markierungsblende 32 ist auf der Innenseite einer drehbaren Markierungshülse 34 (Fig. 9) so befestigt, so daß sie im Sichtfeld des dargestellten Bildes liegt. Die drehbare Markierungshülse 34 weist auf ihrer Außenseite eine schraubenförmig verlaufende Nut 35 auf, in die ein erster Stift 36 einsteht, wie aus Fig. 9 sowie aus der vergrößerten Detaildarstellung in Fig. 14 ersichtlich ist.

Der erste Stift 36 ist fest mit einem Teflonring 37 verbunden, der drehbar in einer ringförmigen Innennut 38 einer Gleithülse 39 sitzt. Die Gleithülse 39 weist axial zur Innennut 38 beabstandet eine Bohrung 40 auf, in der ein zweiter Stift 41 sitzt, der durch ein zweites Langloch 42 der Führungshülse 18 hindurchsteht, das sich in Längsrichtung des Endoskopschaftes 3 erstreckt. Das obere Ende des zweiten Stiftes 41 mündet in einer Nut 43, die auf der Innenseite des Betätigungselementes 16 ausgebildet ist und sich im proximalen Bereich des Betätigungselementes 16 schraubenförmig erstreckt.

Aufgrund dieses Aufbaus führt eine Drehung des Betätigungselementes 16 relativ zur Führungshülse 18 dazu, daß die Drehbewegung des Betätigungselementes 16 in eine axiale Bewegung des zweiten Stiftes 41 aufgrund des zweiten Langloches 42 umgesetzt wird. Die axiale Bewegung des zweiten Stiftes 41 führt zu einer axialen Bewegung der Gleithülse 39, was aufgrund des ersten Stiftes 36 dann zu einer Drehbewegung der drehbaren Markierungshülse 34 führt. Somit wird synchron mit dem Verschwenken des Umlenkprismas 7 aufgrund einer Drehung des Betätigungselementes 16 die drehbare Markierungshülse 34 und somit die drehbare Markierungsblende 32 relativ zum Optikrohr 10 und somit relativ zur festen Markierungsblende 31 gedreht.

Wenn der Benutzer im Vergleich zu der in Fig. 13 gezeigten Stellung den Endoskopschaft 3 um 90° nach rechts dreht (durch Drehen des Mittelteils 26 relativ zum Hauptteil 19), wird dadurch auch das Zugrohr 15 um 90° nach rechts gedreht, wodurch über den Bolzen 22 das Betätigungselement 16 ebenfalls nach rechts gedreht wird. Dies führt zu einer Drehung der drehbaren Markierungshülse 34 um 90° nach rechts. Da die feste Markierungshülse 33 aufgrund der Drehung des Endoskopschaftes 3 ebenfalls um 90° nach rechts gedreht wird, sieht ein Benutzer beim Betrachten des Bildes die beiden Markierungsblenden 31, 32 in der in Fig. 15 gezeigten Stellung. Der Benutzer kann somit unmittelbar die Drehstellung des Endoskopschaftes von 90° und die Blickrichtung von 10° erfassen.

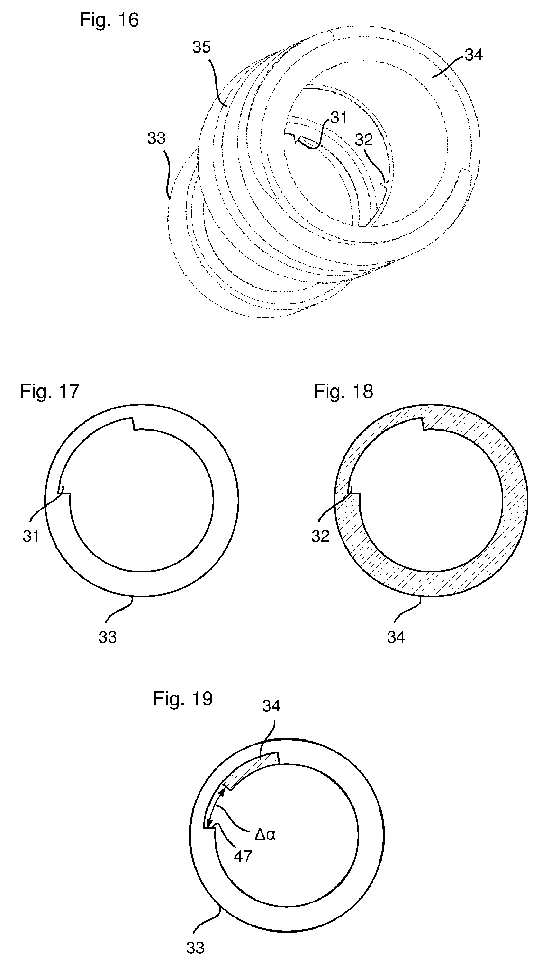

Die beiden Markierungshülsen 33 und 34 sind in Fig. 16 vergrößert in einer perspektivischen Explosionsdarstellung gezeigt. In dieser Darstellung sind die Markierungsblenden 31, 32 deutlich sichtbar.

Die gesamte Mechanik zur Drehung der drehbaren Markierungsblende 32 ist hier als Untersetzungsgetriebe ausgelegt, so daß eine Drehung des Betätigungselementes 16 um einen vorbestimmten Drehwinkel zu einer Drehung der drehbaren Markierungsblende 32 führt, bei der der Drehwinkel kleiner ist als der vorbestimmte Drehwinkel. Die Ausbildung als Untersetzungsgetriebe ist hier gewählt, da die benötigte axiale Verschiebung des Zugrohrs 15 zum Verschwenken des Umlenkprismas 7 einen Drehwinkel des Betätigungselementes 16 erfordert, der größer ist als der gewünschte maximale Drehwinkel der drehbaren Markierungsblende 32 bzw. der drehbaren Markierungshülse 34.

Damit ist es möglich, die Schwenkstellung des Prismas 7 sehr genau einzustellen, da ein relativ großer Drehwinkel des Betätigungselementes 16 notwendig ist, um das Umlenkprisma um einen vorbestimmten Winkel zu verschwenken. Da das Untersetzungsgetriebe so ausgelegt, daß der Winkelabstand der beiden Markierungsblenden 31 und 32 gerade dem Winkel der eingestellten Blickrichtung 5 entspricht, kann trotzdem eine für den Benutzer sinnvolle Darstellung erreicht werden.

Die Außenseite des Betätigungselementes 16 kann ergonomisch ausgebildet sein. So sind in der Darstellung von Fig. 1 als auch in der Darstellung von Fig. 9 Vertiefungen 45 sichtbar, die einen guten Halt gewährleisten.

Wie bereits erwähnt, können die Markierungsblenden 31 und 32 auch andere Formen aufweisen. In Fig. 17 ist eine Draufsicht einer Variante der festen Markierungshülse 33 gezeigt. Die ringabschnittsförmige Ausnehmung dient als feste Markierungsblende 31. In Fig. 18 ist eine Draufsicht auf eine Variante der drehbaren Markierungshülse 34 gezeigt, wobei in gleicher Weise wie bei der festen Markierungshülse 33 die ringabschnittsförmige Ausnehmung die drehbare Markierungsblende 32 bildet. Um die beiden Markierungshülsen in der Darstellung unterscheiden zu können, ist die drehbare Markierungshülse 34 schraffiert dargestellt. Aufgrund der beschriebenen Anordnung der beiden Markierungshülsen 33 und 34 führt eine Verdrehung der drehbaren Markierungshülse 34 relativ zur festen Markierungshülse 33 dazu, daß der Winkelabstand Δα zwischen beiden ringabschnittförmigen Ausnehmungen bzw. zwischen beiden Markierungsblenden 31 und 32 sich ändert (wie in Fig. 19 angedeutet ist). Dieser Winkelabstand Δα zeigt dann für den Betrachter im Bild wiederum die Schwenkstellung des Umlenkprismas 7 an. Die untere Kante 47 zeigt z.B. die Drehstellung des Endoskopschaftes 3 an.

## Patentansprüche

1. Endoskop mit
einem Handgriff (2),
einem mit dem Handgriff (2) verbundenen Endoskopschaft (3),
einer im Endoskopschaft (3) angeordneten Abbildungsoptik (4), die ein in Blickrichtung (5) der Abbildungsoptik (4) vor dem Endoskopschaft (3) befindliches Objekt als Bild abbildet und die eine zur Einstellung der Blickrichtung (5) schwenkbar gelagerte Umlenkeinheit (7, 9) umfaßt, die am vom Handgriff (2) abgewandten distalen Ende des Endoskopschaftes (3) angeordnet ist und deren möglicher Schwenkbereich eine erste und eine zweite Grenze aufweist,
einem am Handgriff (2) angeordneten Betätigungselement (16), das mit der Umlenkeinheit (7, 9) mechanisch gekoppelt ist und mit dem die Schwenkstellung der Umlenkeinheit (7, 9) innerhalb des Schwenkbereiches geändert werden kann, um eine gewünschte Blickrichtung (5) einzustellen,
sowie mit einem verschiebbar im Endoskopschaft (3) angeordneten Schiebeelement (15), dessen proximales Ende mit dem Betätigungselement (16) gekoppelt ist,
wobei eine Betätigung des Betätigungselementes (16) zu einem Verschieben des Schiebeelementes (15) führt und ein erster mechanischer Anschlag (61, 61') vorgesehen ist, an dem die Umlenkeinheit (7, 9) bei Erreichen der ersten Grenze anliegt,
**dadurch gekennzeichnet, dass**
der erste Anschlag (61, 61') durch das distale Ende des Schiebeelements (15) gebildet ist.

2. Endoskop nach Anspruch 1, **dadurch gekennzeichnet, dass** ein zweiter mechanischer Anschlag (62) vorgesehen ist, an dem die Umlenkeinheit (7, 9) bei Erreichen der zweiten Grenze anliegt.

3. Endoskop nach Anspruch 2, **dadurch gekennzeichnet, dass** der zweite Anschlag (62) an einer Fassung (64) eines zur Umlenkeinheit (7, 9) benachbarten optischen Elementes (8) der Abbildungsoptik (4) ausgebildet ist.

4. Endoskop nach einem der obigen Ansprüche, **dadurch gekennzeichnet, dass** das Schiebeelement (15) als Rohr ausgebildet ist.

5. Endoskop nach einem der obigen Ansprüche, **dadurch gekennzeichnet, dass** das distale Ende des Schiebeelementes (15) einen Abschirmungsabschnitt (51) aufweist, wobei das distale Ende des Schiebeelementes (15) so mit der Umlenkeinheit (7, 9) gekoppelt ist, daß eine Verschiebung des Schiebeelements (15) eine Änderung der Schwenkstellung der Umlenkeinheit (7,9) und gleichzeitig eine Verschiebung des Abschirmungsabschnittes (51) bewirkt, um zeitgleich mit der Einstellung der gewünschten Blickrichtung eine Streulichtabschirmung für den Bereich (50) zwischen der Umlenkeinheit (7, 9) und einem der Umlenkeinheit (7, 9) nachfolgenden Optikelement (8) der Abbildungsoptik (4) zu erreichen.

6. Endoskop nach Anspruch 5, **dadurch gekennzeichnet, dass** das distale Ende des Schiebeelementes (15) zum Verschwenken der Umlenkeinheit (7, 9) an einem Bereich angreift, der nicht auf der Schwenkachse der Umlenkeinheit (7, 9) liegt.

7. Endoskop nach Anspruch 5 oder 6, **dadurch gekennzeichnet, dass** zur Kopplung des distalen Endes des Schiebeelementes (15) mit der Umlenkeinheit (7, 9) ein Betätigungszapfen (13) als erstes Verbindungselement und eine den Betätigungszapfen (13) führende Aufnahme (14) als zweites Verbindungselement vorgesehen sind, wobei eines der beiden Verbindungselemente an der Umlenkeinheit (7,9) und das andere der beiden Verbindungselemente am Schiebeelement (15) vorgesehen ist.

8. Endoskop nach einem der obigen Ansprüche, **dadurch gekennzeichnet, dass** die Umlenkeinheit (7, 9) ein in einem Halter (9) befestigtes Umlenkelement (7) aufweist, wobei ein Teil des Halters (9) beim Erreichen der ersten oder zweiten Grenze am entsprechenden Anschlag (61, 61', 62) anliegt.

9. Endoskop nach Anspruch 8, **dadurch gekennzeichnet, dass** das Umlenkelement (7) als Prisma ausgebildet ist.

## Claims

1. Endoscope with
a handle (2),
an endoscope shaft (3) connected to the handle (2),
imaging optics (4) arranged in the endoscope shaft (3) which image an object located in the direction of view (5) of the imaging optics (4) in front of the endoscope shaft (3) as an image and which to set the desired direction of view (5) comprise a swivellably housed deflecting unit (7, 9) which is positioned at the distal end of the endoscope shaft (3) facing away from the handle (2) and the possible swivel range of which has a first and a second limit,
an actuating element (16) which is attached to the handle (2) and which is mechanically connected to the deflecting unit (7, 9) and with which the swivel position of the deflecting unit (7, 9) within the swivel range can be changed in order to set a desired direction of view (5),
as well as a sliding element (15) which is arranged displaceable in the endoscope shaft (3), the proximal end of the sliding element (15) is connected to the actuating element (16),
wherein an actuation of the actuating element (16) leads to a displacement of the sliding element (15) and a first mechanical stop (61, 61') is provided against which the deflecting unit (7, 9) lies when the first limit is reached
**characterized in that**
the first stop (61, 61') is formed by distal end of the sliding element (15).

2. Endoscope according to claim 1, **characterized in that** a second mechanical stop (62) is provided against which the deflecting unit (7, 9) lies when the second limit is reached.

3. Endoscope according to claim 2, **characterized in that** the second stop (62) is formed at a mount (64) of an optical element (8) adjacent to the deflecting unit (7, 9) of the imaging optics (4).

4. Endoscope according to one of the above claims, **characterized in that** the sliding element (15) is formed as a tube.

5. Endoscope according to one of the above claims, **characterized in that** the distal end of the sliding element (15) has a screening section (51), wherein the distal end of the sliding element (15) is connected to the deflecting unit (7, 9) such that a displacement of the sliding element (15) brings about a change in the swivel position of the deflecting unit (7, 9) and simultaneously a displacement of the screening section (51), in order to achieve, at the same time as setting the desired direction of view, a scattered-light screening for the area (50) between the deflecting unit (7, 9) and an optical element (8) following the deflector (7, 9) of the imaging optics (4).

6. Endoscope according to claim 5, **characterized in that**, to swivel the deflecting unit (7, 9), the distal end of the sliding element (15) engages in an area which does not lie on the swivel axis of the deflecting unit (7, 9).

7. Endoscope according to claim 5 or 6, **characterized in that**, to connect the distal end of the sliding element (15) to the deflecting unit (7, 9) an actuating pin (13) is provided as first connecting element and a receptacle (14) guiding the actuating pin (13) as second connecting element, wherein one of the two connecting elements is provided at the deflecting unit (7, 9) and the other of the two connecting elements at the sliding element (15).

8. Endoscope according to one of the above claims, **characterized in that** the deflecting unit (7, 9) has a deflecting element (7) fixed in a holder (9), wherein, when the first or second limit is reached, a part of the holder (9) lies against the corresponding stop (61, 61', 62).

9. Endoscope according to claim 8, **characterized in that** the deflecting element (7) is formed as a prism.

## Revendications

1. Endoscope avec
une poignée (2),
une tige d'endoscope (3) reliée à une poignée (2),
une optique de reproduction (4) disposée dans une tige d'endoscope (3), qui reproduit un objet se trouvant devant la tige d'endoscope (3), dans la direction du regard (5) de l'optique de reproduction (4), sous la forme d'une image et qui comprend une unité de renvoi (7, 9) logée de manière pivotante pour le réglage de la direction de regard (5), qui est disposée au niveau d'une extrémité distale de la tige d'endoscope (3), opposée à la poignée (2) et dont la zone de pivotement possible présente une première et une deuxième limite,
un élément d'actionnement (16), disposé au niveau de la poignée (2), qui est couplé mécaniquement avec l'unité de renvoi (7, 9) et avec lequel la position de pivotement de l'unité de renvoi (7, 9) peut être modifiée à l'intérieur de la zone de pivotement, afin de régler une direction de regard (5) souhaitée,
ainsi qu'un élément coulissant (15) disposé de manière coulissante dans la tige d'endoscope (3), dont l'extrémité proximale est couplée à l'élément d'actionnement (16),
un actionnement de l'élément d'actionnement (16) provoquant un coulissement de l'élément coulissant (15) et une première butée mécanique (61, 61') étant prévue, contre laquelle l'unité de renvoi (7, 9) s'appuie lorsque la première limite est atteinte,
**caractérisé en ce que**
la première butée (61, 61') est constituée de l'extrémité distale de l'élément coulissant (15).

2. Endoscope selon la revendication 1, **caractérisé en ce qu'**une deuxième butée mécanique (62) est prévue, contre laquelle l'unité de renvoi (7, 9) s'appuie lorsque la deuxième limite est atteinte.

3. Endoscope selon la revendication 2, **caractérisé en ce que** la deuxième butée (62) est disposée sur une monture (64) d'un élément optique (8), adjacent à l'unité de renvoi (7, 9), de l'optique de reproduction (4).

4. Endoscope selon l'une des revendications précédentes, **caractérisé en ce que** l'élément coulissant (15) est conçu sous la forme d'un tube.

5. Endoscope selon l'une des revendications précédentes, **caractérisé en ce que** l'extrémité distale de l'élément coulissant (15) comprend une portion de protection (51), l'extrémité distale de l'élément coulissant (15) étant couplée à l'unité de renvoi (7, 9) de façon à ce qu'un coulissement de l'élément coulissant (15) provoque une modification de la position de pivotement de l'unité de renvoi (7, 9) et simultanément un coulissement de la portion de protection (51), afin d'obtenir, en même temps que le réglage de la direction de regard souhaitée, une protection contre la lumière diffusée pour la zone (50) entre l'unité de renvoi (7, 9) et un élément optique (8) de l'optique de reproduction (4) suivant l'unité de renvoi (7, 9).

6. Endoscope selon la revendication 5, **caractérisé en ce que** l'extrémité distale de l'élément coulissant (15) s'emboîte, pour le pivotement de l'unité de renvoi (7, 9), au niveau d'une zone qui ne se trouve pas sur l'axe de pivotement de l'unité de renvoi (7, 9).

7. Endoscope selon la revendication 5 ou 6, **caractérisé en ce que**, pour le couplage de l'extrémité distale de l'élément coulissant (15) avec l'unité de renvoi (7, 9), sont prévus un tourillon d'actionnement (13), en tant que premier élément de liaison, et un logement (14) guidant le tourillon d'actionnement (13), en tant que deuxième élément de liaison, un des deux éléments de liaison étant prévu sur l'unité de renvoi (7, 9) et l'autre des deux éléments de renvoi étant prévu sur l'élément coulissant (15).

8. Endoscope selon l'une des revendications précédentes, **caractérisé en ce que** l'unité de renvoi (7, 9) comprend un élément de renvoi (7) fixé dans un support (9), une partie du support (9) s'appuyant contre la butée (61, 61', 62) correspondante lorsque la première ou la deuxième limite est atteinte.

9. Endoscope selon la revendication 8, **caractérisé en ce que** l'élément de renvoi (7) est conçu comme un prisme.
